# EUROPEAN PATENT APPLICATION

(11) **EP 4 084 009 A1**
(43) Date of publication of application: **02.11.2022**
(21) Application number: 21171460.5
(22) Date of filing: 30.04.2021
(51) Int. Cl.: G16H 30/20, G16H 30/40, G06T 7/00, G16H 50/20

(54) **DIAGNOSTIC IMAGING SYSTEM TO SUPPORT A CLINICAL ENDPOINT**

(71) Applicant: Koninklijke Philips N.V., 5656 AG Eindhoven (NL)
(72) Inventor: PEZZOTTI, Nicola, 5656 AE Eindhoven (NL); VAN SLOUN, Ruud Johannes Gerardus, 5656 AE Eindhoven (NL); GRASS, Michael, 5656 AE Eindhoven (NL)
(74) Representative: Philips Intellectual Property & Standards

(57) **Abstract**

The present disclosure relates to a method comprising determining acquisition parameters of an imaging system using a first machine learning model for acquiring image data from a target volume according to the determined acquisition parameters. The acquired data may be used for providing by a second machine learning model data descriptive of at least one clinical endpoint, wherein the first machine learning model is configured to provide acquisition parameters based on outputs of the second machine learning model.

## Description

### FIELD OF THE INVENTION

The invention relates to scanning imaging systems, in particular to a diagnostic imaging system to support a clinical endpoint.

### BACKGROUND OF THE INVENTION

Deep Learning based solutions can be used for reconstructing heavily undersampled magnetic resonance (MR) data. Since less data has to be acquired in a scanner, the examination time can be greatly reduced. Another application is the denoising of MR scans or motion compensated acquisition. At the same time, Deep Learning is becoming increasingly used for medical image analysis, with several automated solutions already deployed in hospitals. However, there is continuous need to improve the implementation of such models.

### SUMMARY OF THE INVENTION

Various embodiments provide for a medical analysis system, method, and computer program product, as described by the subject matter of the independent claims. Advantageous embodiments are described in the dependent claims.

Embodiments of the invention may provide for a diagnostic imaging system, in particular a magnetic resonance examination system comprising an artificial intelligence (AI)/Deep Learning module to control acquisition of image data, reconstruct and analyze diagnostic images. The diagnostic imaging system is provided with a jointly optimized acquisition, reconstruction and diagnosis function that is adapted to a pre-determined clinical endpoint. In particular the diagnostic imaging system may include an adaptive acquisition mechanism that is informed by the current confidence of the prediction, a reconstruction model that provides the input for the diagnostic model, one or more diagnostic models that provide an answer to a clinical question and an uncertainty estimation module that verifies the confidence of the prediction and steer the acquisition accordingly.

A clinical endpoint (which may also be referred to as endpoint) may be a measure of a clinical outcome of an event. For example, the clinical endpoint may enable to assess the benefit or harm of a treatment. The clinical endpoint may refer to an aspect of a patient's clinical or health status that indicates how a patient feels, functions or survives. For example, the clinical endpoint may indicate an occurrence of disease, symptom. Common types of endpoints may include: mortality, morbidity events, symptoms such as pain and itching, function such as Health Assessment Questionnaire Disability Index and health-related quality of life.

Imaging systems involving nuclear imaging techniques such as positron emission tomography (PET), computed tomography (CT) and MRI systems may offer the sensitivity required to image, and thus measure, specific endpoints. For example, the clinical endpoint may be assessed based on PET and MR images. However, clinical assessments may be prone to subjective and bias depending on examiners that analyze the acquired images. Providing predictions and their probabilities according to the present subject matter may improve decisions made by the radiologist. In addition, the determination of a clinical endpoint involves a chain of different processes whose individual optimization is not necessarily a guarantee that the determined endpoint is accurate. The present subject matter may solve this issue by providing a systematic and accurate determination of the clinical endpoints using imaging systems. In particular, the present subject matter may connect an active acquisition to a clinical endpoint as the imaging system may be configured to actively scan to detect anomalies/disease instead of executing an a-priori defined protocol.

In one aspect, the invention relates to a method for determining at least one clinical endpoint for a target volume using an imaging system and a machine learning based clinical endpoint module. The machine learning based clinical endpoint module comprises a first machine learning model that is configured to predict acquisition parameters for acquisition of data by the imaging system and at least one second machine learning model that is configured to predict respectively the at least one clinical endpoint from data acquired according to the acquisition parameters, wherein the first machine learning model is configured to provide/predict acquisition parameters based on outputs of the second machine learning model. The imaging system may, for example, be used for scanning or imaging the target volume in a subject in order to acquire the data. The method comprises:
determining acquisition parameters of the imaging system using the first machine learning model for acquiring image data from the target volume according to the determined acquisition parameters and using the acquired data by the second machine learning model for providing endpoint data indicative of the at least one clinical endpoint. The endpoint data may provide intermediate results that may subsequently be used by a radiologist r, for example, to assign the endpoint data to a particular clinical picture of the at least one clinical endpoint, etc. The radiologist may, for example, use the provided endpoint data for performing a diagnosis or assign the endpoint data to a particular clinical picture, etc.

Each model of the first machine learning model and the at least one second machine learning model may be a trained model, that is the determination of the at least one clinical endpoint is performed by inference of the first machine learning model and the at least one second machine learning model. The first machine learning model may, for example, be a deep neural network. Each of the at least one second machine learning model may be a deep neural network.

The first machine learning model may be configured to receive a first input and to predict the acquisition parameters. The first input may, for example, be acquired image data of the target volume such as k-space data or an intermediate reconstructed image of said acquired data. Additionally, or alternatively, the first input may comprise an output of the at least one second machine learning model. The output may be an uncertainty of the prediction of the at least one second machine learning model or other outputs such as class activation maps e.g., the uncertainty or accuracy of prediction of multiple second machine learning models may be combined to provide the output.

Each of the at least one second machine learning model may receive as input the data acquired (e.g., acquired according to the predicted acquisition parameters) and to predict the respective clinical endpoint. In another example, each of the at least one second machine learning model may receive as input the image reconstructed from data acquired and to predict the respective clinical endpoint. The uncertainty of the prediction of the second machine learning model may be estimated. For example, the uncertainty may be estimated using a Monte Carlo (MC) dropout of the deep neural network. The predicted clinical endpoint may be provided in association with an uncertainty of the prediction so that the radiologist may decide whether the predicted clinical endpoint can be assigned to the case or not.

Each clinical endpoint of the at least one clinical endpoint may be predicted by a respective second machine learning model of the at least one second machine learning model. The at least one clinical endpoint may, for example, comprise a diagnosis, therapy selection, therapy device selection, hospital stay prediction, ICU discharge prediction, outcome prediction, quality of life prediction or combinations thereof.

The machine learning based clinical endpoint module may enable an endpoint chain that starts by controlling acquisition and ends with a prediction of clinical endpoints. In one example, the at least one clinical endpoint may comprise multiple clinical endpoints. In this case, the at least one second machine learning model may comprise multiple second machine learning model for predicting the multiple clinical endpoints respectively. This may be advantageous as it may enable to use a same endpoint chain for determining different clinical endpoints. In another example, the at least one clinical endpoint may be a single clinical endpoint. In this case, the at least one second machine learning model may comprise a single second machine learning model for predicting the single clinical endpoint. This may be advantageous as the optimization of the endpoint chain for a single clinical endpoint may provide more accurate results.

The fact that the first machine learning model is configured to provide acquisition parameters based on outputs of the second machine learning model may mean that during model inference at an inference iteration k+1, the first machine learning model may receive an output (from iteration k) of the second machine learning model such as class activation maps or the prediction uncertainty and may use that output (and optionally the acquired data in addition) of the iteration k to predict acquisition parameters for acquiring data in the current iteration k+1. That is, the first machine learning model may have inferred 'what to do' from analysis of endpoint predictions by the second machine learning model (e.g., via class activation maps, uncertainty estimates). In this case, the first machine learning model and second machine learning model may independently be trained. For example, the first machine learning model may be trained using output (e.g., class activation maps) of the second machine learning model as input and associated acquisition parameters as labels or as values to regress in order to predict the acquisition parameters e.g., according to classification and regression tasks. The second machine learning model may be trained using as input the acquired data or image reconstructed from the acquired data and a true endpoint associated with the input as a label or as value to regress.

Alternatively, the first machine learning model is configured to provide acquisition parameters based on outputs of the second machine learning model because the first machine learning model and second machine learning model are jointly trained or optimized. In this case, the first machine learning model is configured to receive as input the acquired data, without the output of the second machine learning model because the output of the second machine learning model is taken into account during the joint training.

Therefore, the prediction of the acquisition parameters according to the present subject matter may be performed with an aim/intent to improve the output of the second machine learning model.

The term "machine learning" (ML) refers to a computer algorithm used to extract useful information from training data sets by building probabilistic models (referred to as machine learning models or "predictive models") in an automated way. Machine learning algorithms build a mathematical model based on sample data, known as "training data", in order to make predictions or decisions. The machine learning may be performed using a learning algorithm such as supervised or unsupervised learning, [clustering, classification, linear regression,] reinforcement algorithm, self-learning, etc., etc. A "model" or "predictive model" may for example be a data structure or program such as a neural network, a support vector machine, a decision tree, a Bayesian network etc. The model is adapted to predict an unmeasured value (e.g., which tag corresponds to a given token) from other, known values and/or to predict or select an action to maximize a future reward.

According to one embodiment, the determining of the acquisition parameters is performed during acquisition of data by the imaging system comprises: acquiring data (named DATA) by the imaging system; inputting the acquired data to the first machine learning model for determining the acquisition parameters. If the first machine learning model is not trained jointly with the second machine learning model, the inputting step may alternatively or additional provide as input a previous output (e.g., a prediction uncertainty) of the second machine learning model that is obtained from said acquired data DATA. This embodiment may enable an online/real-time/active adaptation of the physical acquisition to a given clinical endpoint using machine learning. This may enable to connect the active acquisition to a clinical endpoint for enabling a system that actively 'scans' to detect anomalies/disease instead of executing an a-priori defined protocol.

According to one embodiment, the determining of the acquisition parameters is iteratively performed by the first machine learning model using acquired data and/or output of the second machine learning model of a previous iteration as input of the first machine learning model for a current iteration. This embodiment may enable an iterative process. The iterative process refers to the act of updating (during the execution/inference phase) the output of the first machine learning model based on the prior acquired data. For example, OUT1[k+1] = ML1(f(data[k])), where ML1 is the first machine learning model, k is the iteration step, data[k] is the data currently available (acquired thus far), f(.) some function which can be the second machine learning model, or some uncertainty predictor, or simply a function passing through the data (i.e. f(.) enables to provide as input acquired data or output of ML2); OUT1[k+1] are the new acquisition parameters, which are used to then acquire data[k+1] and so on.

According to one embodiment, the first machine learning model is configured to provide acquisition parameters based on outputs of the second machine learning model comprises:
training the machine learning based clinical endpoint module by jointly training the first machine learning model and second machine learning model, the training being performed using a training dataset comprising inputs of the first machine learning based model and endpoints corresponding to the second machine learning based model, the training comprising optimizing an objective function for improving accuracy of the prediction of the endpoint. The objective function may be optimized to further reduce prediction uncertainty of the prediction of the clinical endpoint.

The training set for the joint training may, for example, comprise the first input of the first machine learning model and clinical endpoints as labels or values to regress. The first input may, for example, be acquired image data of the target volume such as k-space data or an intermediate reconstructed image of said acquired data. Additionally, or alternatively, the first input may comprise an output of the second machine learning model. The output may be an uncertainty of the prediction of the second machine learning model or other outputs such as class activation maps.

Given the input data of the first machine learning model, the task of jointly training the first and second machine learning models may be to increase accuracy of the prediction and reduce the uncertainty of the prediction by the second machine learning model. The joint training may be performed by joint optimization by gradient descent. For example, gradient descent may be applied to update the parameters of the first and second machine learning models based on an objective function. For example, the gradient descent scheme may be interpreted as a back-propagation algorithm on a new neural network which unifies the first and second machine learning models.

In another example, the first machine learning model may be configured to provide acquisition parameters based on outputs of the second machine learning model without having to be jointly trained with the second machine learning model. For that, the first machine learning model may be independently trained using outputs of the second machine learning model as input and true acquisition parameters as labels or as values to regress.

According to one embodiment, the acquisition parameters comprise an amount of acquired data. The at least one clinical endpoint comprises two or more clinical endpoints. The training is performed for predicting a first clinical endpoint of the two clinical endpoints using a first amount of the acquired data and the respective second machine learning model and for predicting the second clinical endpoint with a different second amount of the acquired data using the respective second machine learning model. For example, the presence of a hemorrhagic stroke can be detected with confidence after only a limited number of k-space lines are acquired and can be produced as a clinical outcome, while more subtle outcomes may be produced after more data is acquired.

According to one embodiment, the machine learning based clinical endpoint module comprises a third machine learning model for reconstructing an image from the data acquired by the imaging system according to the predicted acquisition parameters. In this case, the training of the machine learning based clinical endpoint module results in a trained third machine learning model by jointly training the first, second and third machine learning models.

This embodiment may enable a reconstruction and analysis pipeline that integrates AI based acquisition, reconstruction and analysis which is jointly optimized. The pipeline may provide a solution for a clinical endpoint, e.g., the detection and classification of strokes. In this case, the system may not produce an image only, but a specific diagnosis. This is achieved by integrating the medical image analysis into the reconstruction pipeline. Since the whole pipeline is optimized for the clinical endpoint, the advantage of this approach may be that the system can be optimized, hence with lower specifications, both hardware and software. For example, a low-field MR system can be portable or used in the emergency room to quickly detect strokes in patients.

In one example, the reconstruction may be iterative and clinical outcomes are produced at different iterations based on the confidence in the prediction (uncertainty). For example, the presence of a hemorrhagic stroke can be detected with confidence after only a limited number of k-space lines are acquired and can be produced as a clinical outcome, while more subtle outcomes are produced after more data is acquired.

According to one embodiment, the training is performed with backpropagation of a value indicative of the accuracy to each of the machine learning models of the machine learning based clinical endpoint module. According to one embodiment, the training is performed with backpropagation of a value indicative of the prediction uncertainty and the accuracy to each of the machine learning models of the machine learning based clinical endpoint module.

According to one embodiment, the imaging system is an MRI system, wherein the acquisition parameters comprise sampling parameters for sampling k-space, wherein the acquired data comprises k-space data sampled according to the sampling parameters.

According to one embodiment, the imaging system is a CT imaging system, wherein the acquisition parameters comprise angular sampling parameters and radiation dose parameters.

According to one embodiment, the imaging system is a PET imaging system, wherein the acquisition parameters comprise parameters for the tracer and acquisition time per bed position.

According to one embodiment, the imaging system is an ultrasound imaging system, wherein the acquisition parameters comprise parameters for transmission and/or acquisition of ultrasound signals.

According to one embodiment, the first machine learning model is a reinforcement learning model or a probabilistic sampling model.

In a different embodiment of this invention, the system predicts based on diagnostic data, a therapy or device to be used to guarantee the best outcome or highest quality of life. To this end, the whole machine learning based clinical endpoint module may be trained against the outcome data, rather-the-then/in-combination-with the diagnosis for the specific clinical endpoint.

In another aspect, the invention relates to a computer program product comprising machine executable instructions for execution by a processor, wherein execution of the machine executable instructions causes the processor to the methods of any of the preceding embodiments.

In one aspect, the invention relates to diagnostic imaging system (or medical analysis system) for imaging systems, the diagnostic imaging system comprising a machine learning based clinical endpoint module, the machine learning based clinical endpoint module comprising a first machine learning model that is configured to predict acquisition parameters for acquisition of data by the imaging system and at least one second machine learning model that is configured to predict respectively the at least one endpoint from data acquired according to the acquisition parameters, wherein the first machine learning model is configured to provide acquisition parameters based on outputs of the second machine learning model.

In one aspect, the invention relates to a medical analysis system for predicting at least one clinical endpoint for a target volume using an imaging system, the medical analysis system comprising a processor and at least one memory storing machine executable instructions, the medical analysis system comprising a machine learning based clinical endpoint module, the machine learning based clinical endpoint module comprising a first machine learning model that is configured to predict acquisition parameters for acquisition of data by the imaging system and at least one second machine learning model that is configured to predict respectively the at least one endpoint from data acquired according to the acquisition parameters, wherein the first machine learning model is configured to provide acquisition parameters based on outputs of the second machine learning model, the processor being configured for controlling the medical analysis system, wherein execution of the machine executable instructions causes the processor to: determine/predict acquisition parameters of the imaging system using the first machine learning model for acquiring image data by the imaging system from the target volume according to the determined acquisition parameters; predict the at least one endpoint using the acquired data and the second machine learning model. For example, the execution of the machine executable instructions may cause the processor to provide an input to the first machine learning model to predict acquisition parameters, control the imaging system to acquire the image data according to the determined acquisition parameters, receive said acquired image data from the imaging system, and to provide said received acquired image data as input to the second machine learning model so that the second machine learning model predicts the at least one clinical endpoint using the acquired image data.

It is understood that one or more of the aforementioned embodiments of the invention may be combined as long as the combined embodiments are not mutually exclusive.

### BRIEF DESCRIPTION OF THE DRAWINGS

In the following preferred embodiments of the invention will be described, by way of example only, and with reference to the drawings in which:
Fig. 1 is a schematic diagram of a control system in accordance with the present subject matter,
Fig. 2 is a schematic diagram of a machine learning based clinical endpoint module in accordance with the present subject matter,
Fig. 3 is a flowchart of a method for determining clinical endpoints in accordance with an example of the present subject matter,
Fig. 4 shows a cross-sectional and functional view of an MRI system.

### DETAILED DESCRIPTION OF THE EMBODIMENTS

In the following, like numbered elements in the figures are either similar elements or perform an equivalent function. Elements which have been discussed previously will not necessarily be discussed in later figures if the function is equivalent.

Various structures, systems and devices are schematically depicted in the figures for purposes of explanation only and so as to not obscure the present invention with details that are well known to those skilled in the art. Nevertheless, the attached figures are included to describe and explain illustrative examples of the disclosed subject matter.

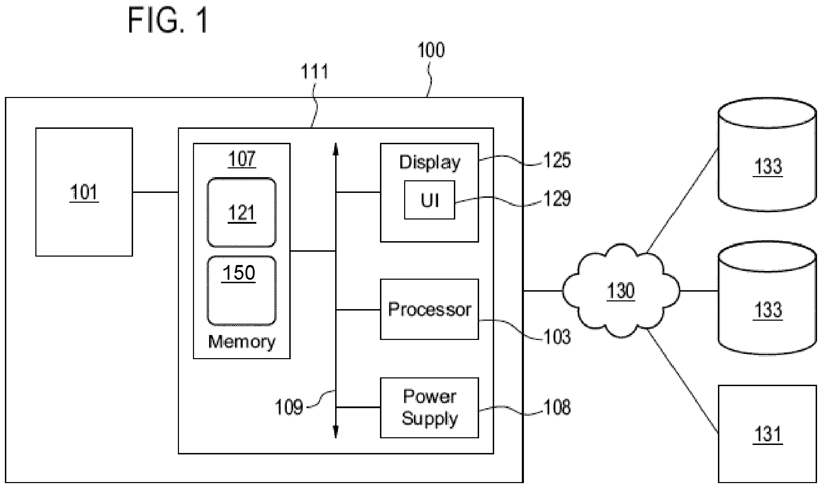

Fig. 1 is a schematic diagram of a medical analysis system 100 (or diagnostic imaging system). The medical analysis system 100 comprises a control system 111 that is configured to connect to an imaging system (or acquisition component) 101. The control system 111 comprises a processor 103, a memory 107 each capable of communicating with one or more components of the medical analysis system 100. For example, components of the control system 111 are coupled to a bidirectional system bus 109.

It will be appreciated that the methods described herein are at least partly non-interactive, and automated by way of computerized systems. For example, these methods can further be implemented in software 121, (including firmware), hardware, or a combination thereof. In exemplary embodiments, the methods described herein are implemented in software, as an executable program, and is executed by a special or general-purpose digital computer, such as a personal computer, workstation, minicomputer, or mainframe computer.

The processor 103 is a hardware device for executing software, particularly that stored in memory 107. The processor 103 can be any custom made or commercially available processor, a central processing unit (CPU), an auxiliary processor among several processors associated with the control system 111, a semiconductor based microprocessor (in the form of a microchip or chip set), a microprocessor, or generally any device for executing software instructions. The processor 103 may control the operation of the scanning imaging system 101.

The memory 107 can include any one or combination of volatile memory elements (e.g., random access memory (RAM, such as DRAM, SRAM, SDRAM, etc.)) and non-volatile memory elements (e.g., ROM, erasable programmable read only memory (EPROM), electronically erasable programmable read only memory (EEPROM), programmable read only memory (PROM). Note that the memory 107 can have a distributed architecture, where various components are situated remote from one another, but can be accessed by the processor 103. Memory 107 may store an instruction or data related to at least one other constituent element of the medical analysis system 100.

The control system 111 may further comprise a display device 125 which displays characters and images and the like e.g., on a user interface 129. The display device 125 may be a touch screen display device.

The medical analysis system 100 may further comprise a power supply 108 for powering the medical analysis system 100. The power supply 108 may for example be a battery or an external source of power, such as electricity supplied by a standard AC outlet.

The imaging system 101 may comprise at least one of MRI, CT and PET-CT imagers. The control system 111 and the imaging system 101 may or may not be an integral part. In other terms, the control system 111 may or may not be external to the imaging system 101.

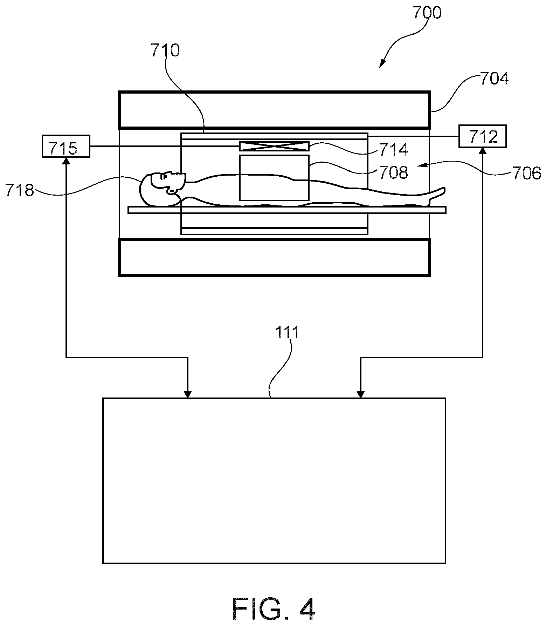

The imaging system 101 comprises components that may be controlled by the processor 103 in order to configure the imaging system 101 to provide image data to the control system 111. The configuration of the imaging system 101 may enable the operation of the imaging system 101. The operation of the imaging system 101 may, for example, be automatic. Fig. 4 shows an example of components of the imaging system 101 being an MRI system.

The connection between the control system 111 and the imaging system 101 may for example comprise a BUS Ethernet connection, WAN connection, or Internet connection etc.

In one example, the imaging system 101 may be configured to provide output data such as images in response to a specified measurement. The control system 111 may be configured to receive data such as MR image data from the imaging system 101. For example, the processor 103 may be adapted to receive information (automatically or upon request) from the imaging system 101 in a compatible digital form so that such information may be displayed on the display device 125. Such information may include operating parameters, alert notifications, and other information related to the use, operation and function of the imaging system 101.

The medical analysis system 100 may be configured to communicate via a network 130 with other scanning imaging systems 131 and/or databases 133. The network 130 comprises for example a wireless local area network (WLAN) connection, WAN (Wide Area Network) connection LAN (Local Area Network) connection or a combination thereof. The databases 133 may comprise information relates to patients, imaging systems, anatomies, scan geometries, scan parameters, scans etc. The databases 133 may for example comprise an electronic medical record (EMR) database comprising patients' EMR, Radiology Information System database, medical image database, PACS, Hospital Information System database and/or other databases comparing data that can be used for planning a scan geometry. The databases 133 may for example comprise training datasets for the training performed by the present subject matter.

The memory comprises a machine learning based clinical endpoint module 150. The machine learning based clinical endpoint module 150 is configured to control acquisition of data, reconstruct of images and determine clinical endpoints. The machine learning based clinical endpoint module 150 may provide diagnostic information of the patient in the system. Using the machine learning based clinical endpoint module 150 may open new applications, such as a low specs system that can be either portable or used in time constrained situations such as the emergency room.

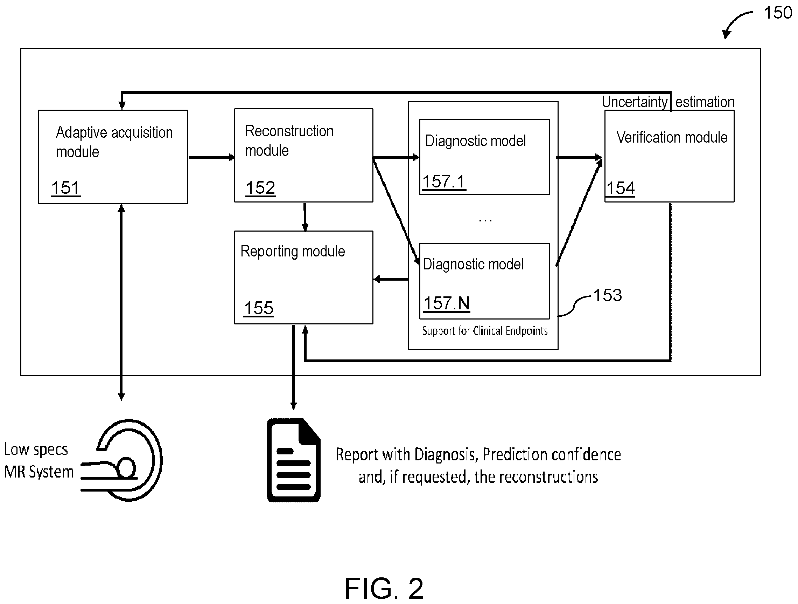

Fig. 2 provides an example of the machine learning based clinical endpoint module 150.

The machine learning based clinical endpoint module 150 may enable an endpoint prediction chain. For that, the machine learning based clinical endpoint module 150 may comprise a prediction chain comprised of an adaptive acquisition module 151, a reconstruction module 152 and a diagnostic module 153. The machine learning based clinical endpoint module 150 may be optimized to provide best performance for the diagnostic module 153.

The diagnostic module 153 may be configured to determine or predict clinical endpoints. The diagnostic module 153 may comprise one or more diagnostic machine learning models 157.1 through 157.N (referred to as ML3). Each of the diagnostic machine learning models 157.1 through 157.N may be configured to predict a respective clinical endpoint such as stroke prediction, bleeding prediction etc. The clinical endpoints may thus be supported by models that can be optimized using a machine learning approach. These models may provide an answer to several diagnostic questions, such as the presence of strokes and their type. Each of the models 157.1 to 157.N may receive as input the reconstruction's output or the acquired data of the imaging system. Each of the models 157.1 to 157.N may be optimized by using a number of examples in which the different types of outcomes are provided.

The reconstruction module 152 may be configured to receive data acquired by the imaging system. The reconstruction module 152 may be used to create an image from the acquired data, and the image is fed as input to the different diagnostic models 157.1 through 157.N of the diagnostic module 153. The present subject matter may be advantageous because the reconstruction module 152 may not be optimized to provide the best Image Quality (IQ), but rather to provide the most relevant information to the diagnostic models 157.1 through 157.N. For example, some level of noise may be often used by the radiologists, but it can hamper the prediction made by the downstream diagnostic models 157.1 to 157.N. However, since the system may be differentiable, the prediction outcome may be optimized efficiently. The reconstruction module 152 may be configured to perform a traditional reconstruction such as SENSE or C-SENSE. In another example, the reconstruction module 152 may comprise a machine learning based reconstruction model (referred to as ML2) or hybrid approaches e.g., combining ML2 with traditional reconstructions. In one example, the reconstruction may be an iterative algorithm. The reconstruction may be, in another example, an unrolled optimization algorithm.

The adaptive acquisition module 151 may be configured to determine acquisition parameters that may be used by the imaging system in order to acquire data. The adaptive acquisition module 151 may set acquisition parameters and communicate them with the imaging system. The adaptive acquisition module 151 may update the acquisition parameters during the acquisition. The adaptive acquisition module 151 may, for example, use an adaptive sampling approach to select which part of k-space to sample by the imaging system. The adaptive sampling may be steered using a coupling mechanism. This is informed by the current uncertainty in the prediction and the acquisition is steered to acquire the most relevant areas. As indicated in Fig. 2, the adaptive acquisition module 151 may be informed by the prediction uncertainty of the diagnostic module 153 e.g., this uncertainty may be a combination of uncertainties of the models 157.1 to 157.N e.g., provided using Ensemble-based methods. The adaptive acquisition module 151 may, for example, be based on reinforcement learning, or on probabilistic sampling. The adaptive acquisition module 151 may be informed by the diagnostic outcome. The adaptive acquisition module 151 may comprise a machine learning model (named ML1) that is configured to predict the acquisition parameters. The input of the machine learning model ML1 may be prior acquired data of the imaging system. The input may, for example, be the (k-space) data acquired thus far in the ongoing acquisition, and possibly presented to ML1 in the form of an intermediate reconstructed image.

The present subject matter may enable a fully differentiable system because the parameters of any of the modules 151 to 153 may be optimized, e.g., denoising strength in the reconstruction, by improving the confidence in the final prediction by the diagnostic module 153. For example, the machine learning model ML1 may be trained jointly with ML2 and the diagnostic models ML3 in an endpoint prediction chain. In another example, the machine learning model ML1 may infer what to do by "analysis" of ML3's processing without joint training. The analysis may, for example, be performed by interpreting the class activation maps or output probabilities of the diagnostic models ML3, or via some uncertainty estimator. This may provide a mechanism in which ML1 and ML3 are not jointly trained on some dataset but by which ML1 can improve the output of ML3.

The machine learning based clinical endpoint module 150 may further comprise a verification module 154 and a reporting module 155. The verification and reporting modules may enable to verify the prediction and steer the acquisition accordingly. The verification module 154 may be configured to estimate the uncertainty of the prediction performed by the diagnostic models 157.1 to 157.N. Uncertainty prediction may be based on Dropout-based methods or based on Ensemble-based methods. The reporting module 155 may perform report generation to provide a report as indicated in Fig. 2. The report generation may be based on a fixed template. The report generation may be based on an NLP system. The reconstructions in the report can be enhanced to classification attribution methods on the reconstructed images.

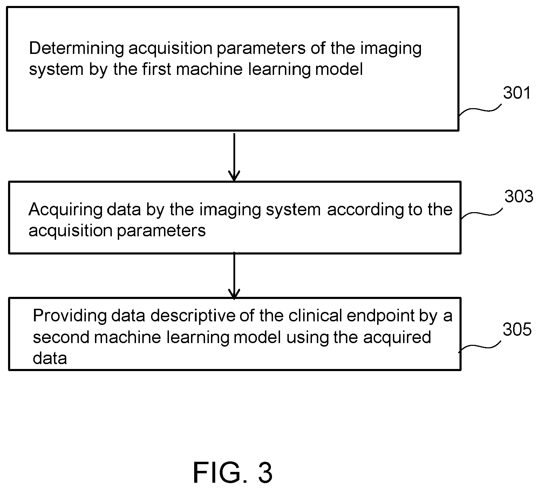

Hence, once trained or optimized the machine learning based clinical endpoint module 150 may be used during acquisition of data as described with reference to Fig. 3.

Fig. 3 is a flowchart of a method for determining clinical endpoints for a target volume according to the present subject matter. Acquisition parameters of the imaging system may be determined using the machine learning model ML1. For that, ML1 may use as input the previous acquired data (e.g., of a last iteration) of the same target volume and/or the output of the one or more models ML3 provided for said acquired data. Data may be acquired in step 303 by the imaging system according to the determined acquisition parameters. The acquired data may be provided in step 305 as input to each of the one or more diagnostic models 157.1 through 157.N in order to predict the respective clinical endpoint.

In one example, steps 303 to 305 may be repeated during acquisition of data by the imaging system. The method of Fig. 3 may be performed in real-time while the data being acquired by the imaging system such as the MRI system.

The image on which the diagnosis is made, may be asked on demand, to validate the prediction made by the system. Moreover, uncertainty estimation techniques may be used to assess whether the prediction made by the system is reliable.

Fig. 4 illustrates a magnetic resonance imaging system 700 as an example of the medical analysis system 100. The magnetic resonance imaging system 700 comprises a magnet 704. The magnet 704 is a superconducting cylindrical type magnet with a bore 706 in it. The use of different types of magnets is also possible; for instance, it is also possible to use both a split cylindrical magnet and a so-called open magnet or a sealed magnet. A split cylindrical magnet is similar to a standard cylindrical magnet, except that the cryostat has been split into two sections to allow access to the iso-plane of the magnet. Such magnets may for instance be used in conjunction with charged particle beam therapy. An open magnet has two magnet sections, one above the other with a space in-between that is large enough to receive a subject 718 to be imaged, the arrangement of the two sections area similar to that of a Helmholtz coil. Inside the cryostat of the cylindrical magnet there is a collection of superconducting coils. Within the bore 706 of the cylindrical magnet 704 there is an imaging zone or volume or anatomy 708 where the magnetic field is strong and uniform enough to perform magnetic resonance imaging.

Within the bore 706 of the magnet there is also a set of magnetic field gradient coils 710 which is used during acquisition of magnetic resonance data to spatially encode magnetic spins of a target volume within the imaging volume or examination volume 708 of the magnet 704. The magnetic field gradient coils 710 are connected to a magnetic field gradient coil power supply 712. The magnetic field gradient coils 710 are intended to be representative. Typically, magnetic field gradient coils 710 contain three separate sets of coils for the encoding in three orthogonal spatial directions. A magnetic field gradient power supply supplies current to the magnetic field gradient coils. The current supplied to the magnetic field gradient coils 710 is controlled as a function of time and may be ramped or pulsed.

MRI system 700 further comprises an RF coil 714 at the subject 718 and adjacent to the examination volume 708 for generating RF excitation pulses. The RF coil 714 may include for example a set of surface coils or other specialized RF coils. The RF coil 714 may be used alternately for transmission of RF pulses as well as for reception of magnetic resonance signals e.g., the RF coil 714 may be implemented as a transmit array coil comprising a plurality of RF transmit coils. The RF coil 714 is connected to one or more RF amplifiers 715.

The magnetic field gradient coil power supply 712 and the RF amplifier 715 are connected to a hardware interface of control system 111. The memory 107 of control system 111 may for example comprise a control module. The control module contains computer-executable code which enables the processor 103 to control the operation and function of the magnetic resonance imaging system 700. It also enables the basic operations of the magnetic resonance imaging system 700 such as the acquisition of magnetic resonance data.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a 'circuit', 'module' or 'system'. Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

A 'computer memory' or 'memory' is an example of a computer-readable storage medium. A computer memory is any memory which is directly accessible to a processor. A 'computer storage' or 'storage' is a further example of a computer-readable storage medium. A computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising 'a processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the 'C' programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device'. A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word 'comprising' does not exclude other elements or steps, and the indefinite article 'a' or 'an' does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical system
- 101: scanning imaging system
- 103: processor
- 107: memory
- 108: power supply
- 109: bus
- 111: control system
- 121: software
- 125: display
- 129: user interface
- 133: databases
- 150: machine learning based clinical endpoint module
- 151: adaptive acquisition module
- 152: reconstruction module
- 153: diagnostic module
- 154: verification module
- 155: reporting module
- 157: diagnostic machine learning models
- 301-305: method steps
- 700: magnetic resonance imaging system
- 704: magnet
- 706: bore of magnet
- 708: imaging zone
- 710: magnetic field gradient coils
- 712: magnetic field gradient coil power supply
- 714: radio-frequency coil
- 715: RF amplifier
- 718: subject

## Claims

1. A medical analysis system for determining at least one clinical endpoint for a target volume using an imaging system, the medical analysis system comprising a processor and at least one memory storing machine executable instructions, the medical analysis system comprising a machine learning based clinical endpoint module, the machine learning based clinical endpoint module comprising a first machine learning model that is configured to predict acquisition parameters for acquisition of data by the imaging system and at least one second machine learning model that is configured to predict respectively the at least one clinical endpoint from data acquired according to the acquisition parameters, wherein the first machine learning model is configured to provide acquisition parameters based on outputs of the second machine learning model, the processor being configured for controlling the medical analysis system, wherein execution of the machine executable instructions causes the processor to:
- determine acquisition parameters of the imaging system using the first machine learning model for acquiring image data by the imaging system from the target volume according to the determined acquisition parameters;
- predict the at least one clinical endpoint using the acquired image data and the second machine learning model.

2. The system of claim 1, wherein execution of the machine executable instructions causes the processor to determine the acquisition parameters during acquisition of data by the imaging system, the determining comprising: inputting the acquired data and/or associated output of the second machine learning model to the first machine learning model for determining the acquisition parameters.

3. The system of claim 1, wherein execution of the machine executable instructions causes the processor to determine the acquisition parameters iteratively using acquired data of a previous iteration and/or output of the second machine learning model of the previous iteration as input of the first machine learning model for a current iteration.

4. The method of claim 1, wherein the first machine learning model is configured to provide acquisition parameters based on outputs of the second machine learning model, upon:
- training the machine learning based clinical endpoint module by jointly training the first machine learning model and second machine learning model,
- the training being performed using a training dataset comprising inputs of the first machine learning based model and endpoints corresponding to the second machine learning based model, the training comprising optimizing an objective function for improving accuracy of the prediction of the clinical endpoint and optionally reducing prediction uncertainty.

5. The system of claim 4, the acquisition parameters comprising an amount of acquired data, the at least one clinical endpoint comprising two or more clinical endpoints, the training being performed for predicting a first clinical endpoint of the two clinical endpoints using a first amount of the acquired data and for predicting the second clinical endpoint with a different second amount of the acquired data.

6. The system of claim 4 or 5, the machine learning based clinical endpoint module comprising a third machine learning model for reconstructing an image from the data acquired by the imaging system according to the predicted acquisition parameters, the training of the machine learning based clinical endpoint module resulting in the third machine learning model.

7. The system of any of the preceding claims 4 to 6, the training being performed with backpropagation of a value indicative of the uncertainty and/or the accuracy to each of the first, second and third machine learning models of the machine learning based clinical endpoint module.

8. The system of any of the preceding claims, the imaging system being a magnetic resonance imaging, MRI, system, wherein the acquisition parameters comprise at least sampling parameters for sampling k-space, wherein the acquired data comprises k-space data sampled according to the sampling parameters.

9. The system of any of the preceding claims 1 to 7, the imaging system being a computed tomography, CT, imaging system, wherein the acquisition parameters comprise at least one of angular sampling parameters, radiation dose parameters and parameters for sampling along the z-axis of a patient having the target volume.

10. The system of any of the preceding claims 1 to 7, the imaging system being a positron emission tomography, PET, imaging system, wherein the acquisition parameters comprise at least one of parameters for the tracer, acquisition time per bed position, and an angular distribution of measured events.

11. The system of any of the preceding claims 1 to 7, the imaging system being an ultrasound imaging system, wherein the acquisition parameters comprise parameters for transmission and/or acquisition of ultrasound signals.

12. The system of any of the preceding claims, the first machine learning model being a reinforcement learning model or a probabilistic sampling model.

13. A method for determining at least one clinical endpoint for a target volume using an imaging system and a machine learning based clinical endpoint module, the machine learning based clinical endpoint module comprising a first machine learning model that is configured to predict acquisition parameters for acquisition of data by the imaging system and at least one second machine learning model that is configured to predict respectively the at least one endpoint from data acquired according to the acquisition parameters, wherein the first machine learning model is configured to provide acquisition parameters based on outputs of the second machine learning model, the method comprising:
- determining acquisition parameters of the imaging system using the first machine learning model for acquiring image data from the target volume according to the determined acquisition parameters;
- using the acquired data by the second machine learning model for providing endpoint data indicative of the at least one clinical endpoint.

14. A computer program product comprising machine executable instructions for execution by a processor, wherein execution of the machine executable instructions causes the processor to perform the method of any of the preceding claims.

15. An imaging system comprising the system of claim 1, the imaging system being configured to acquire the data, wherein the imaging system comprises an MRI system, CT system or PET system.
